Europäisches Patentamt

European Patent Office

Office européen des brevets

(19)

(11) Veröffentlichungsnummer: **0 128 400**
**B1**

(12) **EUROPÄISCHE PATENTSCHRIFT**

(45) Veröffentlichungstag der Patentschrift:
**14.09.88**

(21) Anmeldenummer: **84105739.1**

(22) Anmeldetag: **19.05.84**

(51) Int. Cl.⁴: **C 07 C 31/04, C 07 C 29/15**

(54) **Verfahren zur Herstellung von Methanol.**

(30) Priorität: **25.05.83 DE 3318855**

(43) Veröffentlichungstag der Anmeldung:
**19.12.84 Patentblatt 84/51**

(45) Bekanntmachung des Hinweises auf die Patenterteilung:
**14.09.88 Patentblatt 88/37**

(84) Benannte Vertragsstaaten:
**BE DE FR GB IT NL**

(56) Entgegenhaltungen:
**AT - B - 281 777**
**DE - A - 1 930 003**
**DE - A - 3 203 748**
**DE - B - 2 449 493**
**DE - B - 2 846 614**

**ULLMANNS ENCYCLOPÄDIE DER TECHNISCHEN CHEMIE, 4. Auflage, Band 16, 1978 VERLAG CHEMIE, Weinheim, New York Seiten 627, 628**

(73) Patentinhaber: **BASF Aktiengesellschaft,**
**Carl-Bosch-Strasse 38, D-6700 Ludwigshafen (DE)**
Patentinhaber: **Linde Aktiengesellschaft,**
**Abraham-Lincoln-Strasse 21, D-6200 Wiesbaden (DE)**

(72) Erfinder: **Broecker, Franz Josef, Dr., Schwanthaler Allee 20, D-6700 Ludwigshafen (DE)**
Erfinder: **Dümbgen, Gerd, Dr., Sudetenstrasse 4, D-6701 Dannstadt-Schauernheim (DE)**
Erfinder: **Pies, Wolfgang, Dr., Heidelberger Ring 30a, D-6710 Frankenthal (DE)**
Erfinder: **Schlichthaerle, Gottfried, Dr., Hauberallee 19, D-6730 Neustadt (DE)**
Erfinder: **Weber, Günter, Dr. Dipl.-Chem., Bergerweg 6, D-8157 Linden (DE)**

## Beschreibung

Die Erfindung betrifft ein Verfahren zur Herstellung von Methanol durch Umsetzung von Wasserstoff, Kohlenmonoxid, Kohlendioxid und/oder Wasserdampf in Gegenwart von Kupfer und Zink enthaltenden Katalysatoren.

Es sind derzeit zwei Hauptvarianten zur Herstellung von Methanol aus Synthesegasen, die u.a. Wasserstoff, Kohlenmonoxid, Kohlendioxid und Wasser enthalten, bekannt. Die ältere Verfahrensweise, auch Hochdruckmethanolsynthese genannt, verwendet Katalysatoren auf der Grundlage von Zink- und Chromoxid. Diese Katalysatoren sind unempfindlich gegen eine Reihe von Katalysatorgiften wie z.B. Schwefel und Chlor und zeigen eine hohe Alterungsbeständigkeit. Die Cr/Zn-Katalysatoren sind jedoch nicht sehr reaktiv, sie benötigen hohe Reaktionstemperaturen, beispielsweise 320 bis 380 °C, und wegen der Gleichgewichtslage damit notwendigerweise hohe Reaktionsdrücke wie z.B. 300 bis 340 bar.

Die neuere Verfahrensvariante, auch Niederdruckmethanolsynthese genannt, verwendet kupfer- und zinkhaltige Katalysatoren. Diese Katalysatoren sind wesentlich aktiver, sie erlauben niedrigere Reaktionstemperaturen, z.B. 220 bis 270 °C, und verringern durch die bei niedrigen Temperaturen günstigere Gleichgewichtslage den technischen Aufwand, da diese Synthese bei niedrigeren Drücken, beispielsweise bei 50 bis 100 bar, durchgeführt werden kann.

Nachteilig ist die höhere Neigung der Cu/Znhaltigen Katalysatoren zu desaktivieren. Die Ursachen, die zu einer Alterung der Katalysatoren führen, sind vielfältig und die Klärung der einzelnen Desaktivierungsmechanismen steht noch weitgehend aus. Die Alterung kann z.B. auf einer Verringerung der Zahl der katalytisch aktiven Zentren durch temperaturbedingte Rekristallisation oder durch Blockierung der aktiven Zentren wie beispielsweise durch Reaktion mit schwefel- und chlorhaltigen Katalysatorgiften erfolgen. Weiterhin ist eine Bedeckung der katalytisch aktiven Oberflächen durch andere Stoffe denkbar wie z.B. durch Zersetzungsprodukte von Metallcarbonylen oder auch durch Verbindungen, die aus konkurrierenden Reaktionen hervorgehen. Man hat auch schon Cu- und Zn-haltige bifunktionelle Katalysatoren für die Dimethylether-Synthese, die ausserdem Chrom und/oder hohe Anteile an sauren und dehydratisierenden Komponenten enthalten, mit sauerstoffhaltigen Gasen zu reaktivieren versucht. Die Bildung von Russ oder koksartigen Abscheidungen, wie sie bei der bei Temperaturen über 300 °C durchgeführten Dimethylether-Synthese in Gegenwart von dehydratisierenden Katalysatoren beobachtet wird und die bei dieser Synthese als eine der Hauptursachen der Desaktivierung angesehen werden darf, ist nach allgemeiner Auffassung für die bei niedrigen Temperaturen durchgeführte ND-Methanolsynthese ohne Bedeutung (Ullmann, 4. Auflage, Bd. 16, S. 627).

Man hat auch schon versucht, die Standzeit kupfer- und zinkhaltiger ND-Methanolkatalysatoren durch die Vorreinigung des verwendeten Synthesegases zu erhöhen. Daneben sind zahlreiche Vorschläge bekannt, durch Zugabe von Promotoren die Thermostabilität der Katalysatoren zu erhöhen.

Dennoch bleibt eine nicht unerhebliche Alterung der ND-Methanolkatalysatoren bestehen, die durch keine der bekannten Massnahmen verhindert werden kann und die es notwendig macht, den Katalysator nach einer gewissen Standzeit auszutauschen. Dieses Vorgehen ist nicht nur wegen der Neubeschaffung des Katalysators kostenaufwendig, es ist auch wegen der mit dem Ausbau des gealterten Katalysators und Wiedereinbaus des frischen Kontakts verbundenen Massnahmen technisch aufwendig und zeitraubend. Auch die Regenerierung nach Abnahme der Reaktivität, z.B. durch eine Behandlung mit Sauerstoff, d.h. durch Abbrennen der Koksablagerungen, erforderte die zeitraubenden Massnahmen des Aus- und Wiedereinbaues des zu regenerierenden Katalysators.

Aufgabe der vorliegenden Erfindung ist es daher, ein Verfahren zu entwickeln, das es gestattet, die Folgen der zumindest teilweise methanolsynthesespezifischen Alterung ganz oder teilweise zu beheben und die ursprüngliche Aktivität des Cu/Zn-Kontakts ganz oder zumindest in erheblichem Umfang wieder herzustellen.

Insbesondere war die Aufgabe gestellt, die Verfahrensführung von der Einbringung des Katalysators und Reduktion zum betriebsfertigen Zustand, Kontrolle des Reaktionsablaufs zur Einhaltung optimaler Dauer und Regenerierung aufeinander abzustimmen.

Es wurde nun gefunden, dass man das Verfahren zur Herstellung von Methanol durch katalytische Umsetzung von Synthesegasgemischen, enthaltend Wasserstoff, Kohlenmonoxid, Kohlendioxid und/oder Wasser bei Temperaturen zwischen 200 und 320 °C und Drücken zwischen 30 und 300 bar in adiabatisch und/oder isotherm geführten Reaktoren in Gegenwart eines Kupfer und Zink enthaltenden Katalysators in der gewünschten Weise optimieren kann, wenn der frische Katalysator vor der Inbetriebnahme des Verfahrens zunächst unter Normaldruck oder leichtem Überdruck bei von 150 bis 250 °C ansteigenden Temperaturen mit einem wasserstoffhaltigen Gas reduziert wird, wobei die Reduktion solange fortgesetzt wird, bis die Bildung von Reduktionswasser deutlich nachlässt und dann erst die Synthese unter bekannten Bedingungen eingeleitet und solange fortgeführt wird, bis eine deutliche Abnahme der Methanolbildung in der Reaktionszone eintritt, die Reaktion unterbrochen und der Katalysator anschliessend unmittelbar nach der Unterbrechung der Reaktion mit Inertgasen bei Temperaturen von 10 bis 300 °C gespült und bei normalem oder mässig erhöhtem Druck durch Überleiten von sauerstoffhaltigen Gasen bei Temperaturen von 150 bis 200 °C über den Katalysator in situ regeneriert wird, wobei der Sauerstoffgehalt während des gesamten Verlaufs der Regenerierung der jeweiligen Temperatur angepasst wird und die

Überleitung der sauerstoffhaltigen Gase solange fortgesetzt wird, bis die Spitze des Temperaturprofils den gesamten Katalysator durchschritten hat.

Als Inertgas für die Spülung sind z.B. Stickstoff oder Methan geeignet. Sofern die Spülung des Katalysators mit Methan vorgenommen wird, empfiehlt es sich, vor der Regenerierung auf Stickstoffspülung umzuschalten, um letzte Reste von Methan aus der Katalysatorzone zu entfernen, bevor der zur Regenerierung notwendige Sauerstoff eingeleitet wird.

Bei der Durchführung des Verfahrens wird zweckmässigerweise vor der Inbetriebnahme des Verfahrens der frische Katalysator unter Normaldruck oder leichtem Überdruck, bei einer von 150 bis 250 °C ansteigenden Temperatur durch Überleiten eines wasserstoffhaltigen Gases, z.B. Stickstoff-Wasserstoffgemische oder Stickstoffsynthesegasgemische, reduziert. Zweckmässig wird bei der Inbetriebnahme zunächst die Temperatur von 150 bis 180 °C erhöht und erst bei Nachlassen der Bildung von Reaktionswasser, d.h. nach Ablauf der Hauptreaktion, die Temperatur langsam weitergesteigert, z.B. bis auf 230 °C.

Die Synthese kann dann unter an sich bekannten Bedingungen eingeleitet werden.

Die ND-Methanolsynthese wird bei Drücken von 30 bis 300 bar, vorzugsweise 40 bis 120 bar und bei Temperaturen zwischen 200 und 320 °C, vorzugsweise 230 bis 280 °C, unter nahezu isothermen und/oder adiabatischen Bedingungen durchgeführt. Als Katalysator werden kupfer- und zinkhaltige Katalysatoren mit Kupfergehalten zwischen 8 bis 70 Gew.-% $CuO$, vorzugsweise 15 bis 60 Gew.-% $ZnO$ eingesetzt, die zusätzlich Metallverbindungen der zweiten und dritten Hauptgruppe des Periodensystems wie Magnesium und Aluminium und/oder der dritten bis siebten Nebengruppe wie Lanthan, Thorium, Vanadium, Chrom und Mangan in Mengen von 0 bis 50 Gew.-% Metalloxid enthalten können. Bevorzugt werden Katalysatoren mit 0 bis 40 Gew.-% $Al_2O_3$, vorteilhaft 1 bis 8 Gew.-% $Al_2O_3$ und/oder 0 bis 35 Gew.-% $Cr_2O_3$, vorteilhaft 1 bis 15 Gew.-% $Cr_2O_3$ und/oder 0 bis 15 Gew.-% $V_2O_5$, vorteilhaft 2 bis 10 Gew.-% $V_2O_5$ als Zusatz verwendet. Die Katalysatoren können durch Fällung aus wässrigen Lösungen entsprechender Metallsalze oder aber auch durch Tränken eines im wesentlichen katalytisch inerten Trägers und nachfolgende Trocknung und Calcinierung hergestellt werden. Um Dimethyletherbildung zu vermeiden, ist es zweckmässig, den Katalysatoren keine dehydratisierenden Komponenten beizumischen, z.B. Zeolithe und/oder $\gamma$-$Al_2O_3$. Ein geeigneter Katalysator ist z.B. der in Beispiel 1 beschriebene Cu/Zn/Al-Katalysator der DE-PS 2 846 614.

Der Vorteil des erfindungsgemässen Verfahrens besteht darin, dass man ohne Austausch des Katalysators mit Aus- und Wiedereinfüllen im Reaktor eine weitgehende Reaktivierung erreicht, die je nach Verfahrensführung ein- oder mehrfach wiederholt werden kann und somit insgesamt die Standzeit des Katalysators erheblich verlängert.

Auch bei einer wiederholten Regenerierung wird in der beschriebenen Weise verfahren.

In den nachfolgenden Beispielen wird das erfindungsgemässe Verfahren näher erläutert.

Beispiel 1

Ein ND-Methanolkatalysator mit einem Gehalt von 36 Gew.-% $CuO$, 48 Gew.-% $ZnO$ und 3 Gew.-% $Al_2O_3$, der unter Zusatz von 2 Gew.-% Graphit zu 5 × 5 mm Pillen verpresst wird, wird in einen quasiisotherm betriebenen Rohrreaktor eingebaut und bei 180 °C im geraden Durchgang drucklos mit einem Wasserstoff-Stickstoff-Gemisch (1 Vol.% $H_2$) bei einer Gasbelastung von 300 bis 400 Nl/kg · h reduktiv aktiviert. Der Verlauf der Aktivierung wird über die Bildung des Reduktionswasser verfolgt. Nach Abklingen des Wassergehalts im Reaktoraustrag wird bei gleichbleibender $H_2/N_2$-Zufuhr die Temperatur innerhalb von 2 bis 3 h schrittweise von 180 °C auf 230 °C angehoben und anschliessend der Wasserstoffgehalt des Reaktoreintrittgases innerhalb von 4 bis 5 h von 1% auf 100% gesteigert.

Der Reaktor wird bei 50 bar und 250 °C mit einem metallcarbonylhaltigen Synthesegas (71% $H_2$, 19% CO; 10% $CO_2$) betrieben. Nach mehreren Wochen Betriebszeit sinkt die Aktivität des Katalysators, ausgedrückt als Rohmethanolproduktion, auf 79% des Anfangswertes ab.

Die Regeneration des geschädigten Kontaktes erfolgt nach Spülung des Reaktorsystems mit Stickstoff durch Beaufschlagung des Reaktors mit einem sauerstoffhaltigen Regeneriergas (Stickstoff mit 0,2 bis 0,5% $O_2$) bei Atmosphärendruck im geraden Durchgang. Die Gasbelastung beträgt 3500 Nl/kg · h. Während der Regenerierung wird die Reaktortemperatur zur Vermeidung von Übertemperaturen auf 150 $\pm$ 5 °C konstant gehalten. Nach 24 h wird der Katalysator in der oben beschriebenen Weise erneut reduktiv aktiviert. Nach Beaufschlagung mit dem oben charakterisierten Synthesegas zeigt sich, dass seine Aktivität, gemessen als Rohmethanolauswaage, auf 88% des Anfangswertes angesteigt. Die Desaktivierungsrate des regenerierten Katalysators ist nicht grösser als die des frischen Katalysators. Ausbauproben lassen durch insbesonders in der Gaseintrittszone gegenüber frischem Katalysator erhöhte Eisen- und Nickelgehalte eine Schädigung durch die im Synthesegas als Katalysatorgift enthaltenen Eisen- und Nickelcarbonyle erkennen.

Beispiel 2

Der im Beispiel 1 verwendete Katalysator wird in gleicher Weise reduktiv aktiviert und danach längere Zeit mit einem Synthesegas aus $H_2$, CO und $CO_2$ (molare Zusammensetzung 75:20:5), das geringe Spuren von Katalysatorgiften in Form von Nickelcarbonyl enthält, in einem quasiisothermen Rohrreaktor bei 50 bar und 260 °C beaufschlagt. Nach Absinken der als Rohmethanolauswaage bestimmten Aktivität des Katalysators auf 88% des Anfangswertes wird der Katalysator, wie im ersten Beispiel beschrieben, oxidativ regeneriert, indem er 68 h mit einem $O_2/N_2$-Gemisch (0,2 bis

0,5% $O_2$) bei 150 °C unter Atmosphärendruck behandelt wird. Trotz Entnahme von etwa 3% der Katalysatormasse wird nach der nachfolgenden reduktiven Aktivierung eine gegenüber der Produktion vor Regeneration erhöhte Rohmethanolmenge erzeugt. Sie beträgt 94% des Anfangswertes. Ausbauproben des Katalysators zeigen einen erhöhten Nickelgehalt am Reaktoreingang.

Beispiel 3

Ein technischer ND-Methanolkatalysator mit 36 Gew.-% CuO, 48 Gew.-% ZnO und 3 Gew.% $Al_2O_3$ wird in einem nahezu isotherm betriebenen technischen Reaktor, der Kernstück eines vollständigen Methanolsynthesekreislaufs ist, im Kreislaufbetrieb durch Reduktion in seine aktive Form überführt. Als Reduktionsmittel wird Wasserstoff eingesetzt. Vor Beginn der Aktivierung wird der Kreislauf mit Stickstoff gespült und gefüllt sowie ein Überdruck von 4 bar eingestellt. Das Kreislaufgas wird auf 180 °C aufgewärmt. Die reduktive Aktivierung wird gestartet, indem soviel Wasserstoff in das Kreislaufgas eingespeist wird, dass die $H_2$-Konzentration am Reaktoreingang bei Werten ≤ 0,5 Vol% $H_2$ liegt. Nach dem Anspringen der Reaktion, das durch eine leicht erhöhte Temperatur ($\Delta T < 10$ °C) in der obersten Katalysatorschicht sowie durch das Auftreten von Reduktionswasser anstelle von Wasserstoff im Reaktoraustrittsgas zu erkennen ist, wird die Wasserstoffeinspeisung so gesteigert, dass die theoretisch benötigte $H_2$-Menge innerhalb von einem Tag zugeführt wird und gleichzeitig durch die Wahl der Kreisgasmenge der Wasserstoffgehalt im Reaktoreintrittsgas 1 bis 1,5 Vol.% nicht überschreitet. Der Verlauf der Reduktion wird über die Katalysatorbettemperaturen, die $H_2$- und $H_2O$-Konzentration im Reaktoraustrittsgas und den Anfall von Reduktionswasser kontrolliert. Treten in der durchwandernden Reduktionszone Temperaturspitzen auf, die mehr als 10 °C über der mittleren Bettemperatur liegen, so wird die $H_2$-Einspeisung solange gedrosselt oder unterbrochen, bis diese Temperaturgrenze wieder unterschritten wird. Der Reduktionswasserstoff setzt sich in der Reduktionszone nahezu vollständig zu Reduktionswasser um, das im Reaktoraustrittsgas anstelle des Wasserstoffs erscheint. Während der reduktiven Aktivierung entsteht neben Reduktionswasser auch Kohlendioxid, dessen Anreicherung im Synthesekreislauf durch Ausschleusen von Kreislaufgas auf Werte kleiner 15 Vol.% $CO_2$ gehalten wird.

Die erste Phase der reduktiven Aktivierung ist beendet, wenn

1. die durch eine leicht erhöhte Temperatur gekennzeichnete Reduktionszone das Katalysatorbett durchwandert hat,

2. der Gehalt an Reduktionswasser im Reaktoraustrittsgas abfällt und damit

3. der Wasserstoffgehalt im Reaktoraustrittsgas ansteigt.

Die Nachreduktion, die zur Aktivierung eventuell noch lokal vorhandener nichtreduzierter Katalysatoranteile dient, wird durch eine schrittweise Erhöhung der Katalysatorbettemperatur um 10 bis 20 °C/h auf 220 bis 230 °C eingeleitet. Nach Angleichung der Wasserstoffkonzentration im Reaktoraustrittsgas an die im Reaktoreintrittsgas wird die Wasserstoffeinspeisung so geregelt, dass sich die Wasserstoffkonzentration im Kreislaufgas etwa alle 2 h verdoppelt. Bei Erreichen von 30 Vol.% $H_2$ im Kreisgas ist die Nachreduktionsphase abgeschlossen.

Die Übernahme von Frischgas erfolgt bei 230 °C. Mit dem eingespeisten metallcarbonylfreien Frischgas, das 68 Vol.% $H_2$, 16 Vol.% CO, 12 Vol.% $CO_2$, 0,04 Vol.% $H_2O$ sowie als Rest Inerte ($CH_4$, $N_2$) enthält, wird der Methanolsynthesekreislauf mit 15 bar/h auf einen Reaktionsdruck von 75 bar aufgepresst. Nach dem Einsetzen der Methanolbildung wird die Katalysatorbettemperatur auf 245 °C angehoben. Die Methanolsynthese wird unter diesen Bedingungen, einem Frischgaszu-Reaktoreintrittsgas-Verhältnis von 6 kg/kg und einer Frischgasbelastung von 0,5 $t/t_{Kat} \cdot h$ solange betrieben, bis die spezifische Methanolproduktion auf 80% des Anfangswertes abgesunken ist. Diese Methanolproduktion entspricht einer Katalysatoraktivität von 26% des Anfangszustandes, wenn man die Katalysatoraktivität als Reaktionsgeschwindigkeitskonstante $k_o$ eines kinetischen Modells der Form ( r = Reaktionsgeschwindigkeit)

$$r = k_o \cdot e^{-B/T} \cdot f(P_{H_2}, P_{CO}, P_{CO_2}, P_{CH_3OH})$$

ausdrückt.

Die Reaktion wird dann durch Entspannen des Synthesekreislaufes mit 15 bar/h auf einen Druck von 2 bar und Absenken der Katalysatorbettemperatur auf 220 bis 230 °C unterbrochen. Unmittelbar danach wird der Synthesekreislauf mehrfach mit Stickstoff gespült. Die Spülung erfolgt im Kreislaufbetrieb so, dass Stickstoff in den Synthesekreislauf innerhalb einer Stunde bis auf 10 bar aufgepresst wird und anschliessend im gleichen Zeitraum wieder auf 2 bar entspannt wird. Nach der ersten Spülung wird die Katalysatorbettemperatur auf 170 °C abgesenkt. Die Stickstoffspülung wird solange fortgesetzt, bis Wasserstoff und CO im Kreislaufgas nicht mehr nachweisbar sind (< 1 Vol.%).

Die Regenerierung wird eingeleitet, indem in das mit Stickstoff gefüllte Kreislaufsystem bei 170 °C Luft eingespeist wird. Der Druck beträgt dabei am Reaktoreingang 2 bis 5 bar, der Sauerstoffgehalt des Reaktoreintrittsgases nicht mehr als 0,5 Vol.%. Die Luftmenge beträgt in der Startphase der Regenerierung 2 bis 3 $Nm^3$ Luft/$t_{Kat} \cdot h$. Nach Anspringen der Regenerierung, das durch eine leicht erhöhte Temperatur ($\Delta T \leq 10$ °C) in der obersten Katalysatorschicht zu erkennen ist, wird die Lufteinspeisung innerhalb einer Stunde auf 5 $Nm^3$ Luft/$t_{Kat} \cdot h$ und dann nach einer weiteren Stunde innerhalb einer Stunde auf 10 $Nm^3$ Luft/$t_{Kat} \cdot h$ gesteigert. Der Sauerstoffgehalt im Reaktoreintrittsgas wird nach Anspringen der Reaktion auf Werten kleiner 1 Vol.% gehalten, der des Reaktoraustrittsgases soll Werte von 0,2 Vol.% in den ersten Stunden der Regenerierung nicht überschreiten. Bei Anstieg auf höhere Werte wird in beiden Fällen die Luftzufuhr unterbrochen. Der Verlauf der Regenerierung wird über das Durchlaufen der Reaktionszone mit einer Temperaturerhöhung von $\Delta T \leq 10\,°C$ gegenüber der mittleren Bettemperatur kontrolliert. Treten Temperaturspitzen von $\Delta T > 10\,°C$ auf, so wird die Lufteinspeisung solange gedrosselt oder unterbrochen, bis diese Temperaturgrenze wieder unterschritten wird. Während der Regenerierung wird die Bildung von Kohlendioxid beobachtet. Der Druck des Reaktionssystems wird durch Ausschleusen von Kreislaufgas konstant gehalten (2 bis 5 bar).

Die Hauptphase der Regenerierung ist beendet, wenn

1. die durch eine leicht erhöhte Temperatur gekennzeichnete Reaktionszone das Katalysatorbett durchwandert hat und

2. der Sauerstoffgehalt des Reaktoraustrittsgases mehr als 80% des Sauerstoffgehalts des Reaktoreintrittsgases beträgt.

Zur Regenerierung eventuell lokal noch vorhandener nichtregenerierter Katalysatoranteile wird anschliessend die Lufteinspeisung mit 20 Nm³ Luft/$t_{Kat} \cdot$ h fortgesetzt, bis sich die Sauerstoffkonzentrationen im Reaktoreintrittsgas und Reaktoraustrittsgas angeglichen haben und mindestens 10 Vol.% $O_2$ betragen. Daran anschliessend wird zur Vorbereitung der reduktiven Aktivierung die Katalysatorbettemperatur mit 10°C/h auf 180°C angehoben und gleichzeitig der Synthesekreislauf mit Stickstoff wie oben angegeben gespült, bis der Sauerstoffgehalt im Kreislaufgas auf Werte kleiner 0,2 Vol.% abgesunken ist. Danach wird bei einem Überdruck von 4 bar der regenerierte Katalysator im Kreislaufbetrieb wie oben angegeben reduktiv aktiviert und anschliessend das oben charakterisierte Frischgas in den Synthesekreislauf eingespeist. Die Aktivität des regenerierten Katalysators beträgt nach Wiederanfahren 86% des Anfangswertes mit frischem Katalysator, wenn man die Aktivität in der oben angegebenen Form der Reaktionsgeschwindigkeitskonstanten angibt. Die spezifische Methanolproduktion beträgt dann unter den oben angegebenen Bedingungen 93% des Anfangswertes.

## Patentanspruch

Verfahren zur Herstellung von Methanol durch katalytische Umsetzung eines Synthesegasgemisches, enthaltend Wasserstoff, Kohlenmonoxid, Kohlendioxid und/oder Wasser bei Temperaturen zwischen 200 und 320°C und Drücken zwischen 30 und 300 bar in adiabatisch und/oder isotherm geführten Reaktoren in Gegenwart eines Kupfer und Zink enthaltenden Katalysators, dadurch gekennzeichnet, dass der frische Katalysator vor der Inbetriebnahme des Verfahrens zunächst unter Normaldruck oder leichtem Überdruck bei von 150 bis 250°C ansteigenden Temperaturen mit einem wasserstoffhaltigen Gas reduziert wird, wobei die Reduktion solange fortgesetzt wird, bis die Bildung von Reduktionswasser deutlich nachlässt, und dann erst die Synthese unter bekannten Bedingungen eingeleitet und solange fortgesetzt wird, bis eine deutliche Abnahme der Methanolbildung in der Reaktionszone eintritt, die Reaktion unterbrochen und der Katalysator anschliessend unmittelbar nach der Unterbrechung der Reaktion mit Inertgasen bei Temperaturen von 10 bis 300°C gespült und bei normalen oder mässig erhöhtem Druck durch Überleiten von sauerstoffhaltigen Gasen bei Temperaturen von 150 bis 200°C über den Katalysator in situ regeneriert wird, wobei der Sauerstoffgehalt während des gesamten Verlaufs der Regenerierung der jeweiligen Temperatur angepasst wird und die Überleitung der sauerstoffhaltigen Gase solange fortgesetzt wird, bis die Spitze des Temperaturprofils den gesamten Katalysator durchschritten hat.

## Claim

A process for the preparation of methanol by catalytic conversion of a synthesis gas mixture, containing hydrogen, carbon monoxide, carbon dioxide and/or water, at from 200 to 320°C and under from 30 to 300 bar in adiabatic and/or isothermal reactors in the presence of a catalyst containing copper and zinc, wherein the fresh catalyst is first reduced with a hydrogen-containing gas before the start-up of the process, under atmospheric or slightly superatmospheric pressure at temperatures increasing from 150 to 250°C, the reduction being continued until the formation of water from the reduction reaction substantially declines, and then the synthesis is started under conventional conditions and is continued until the formation of methanol in the reaction zone has declined substantially, after which the reaction is interrupted and immediately thereafter the catalyst is flushed with an inert gas at from 10 to 300°C and is regenerated in situ under atmospheric or moderately superatmospheric pressure by passing an oxygen-containing gas over the catalyst at from 150 to 200°C, the oxygen content being adapted to the particular temperature during the entire course of the regeneration, and the passage of the oxygen-containing gas being continued until the peak of the temperature profile has moved across the entire catalyst.

## Revendication

Procédé de préparation de méthanol par réaction catalytique d'un mélange de gaz de synthèse, contenant de l'hydrogène, de l'oxyde de carbone, de l'anhydride carbonique et/ou de l'eau, à des températures comprises entre 200 et 320°C et sous des pressions comprises entre 30 et 300 bars dans des réacteurs exploités adiabatiquement et/ou isothermiquement, en présence d'un catalyseur contenant du cuivre et du zinc, caractérisé en ce qu'on réduit le catalyseur frais, avant le lancement du procédé, tout d'abord sous la pression normale ou une faible surpression, à des températures croissant de 150 à 250°C, avec un gaz contenant de l'hydrogène, la réduction étant poursuivie jusqu'à ce que la formation d'eau de réduction diminue nettement, puis on ne déclenche qu'alors la synthèse dans des conditions connues et on la continue jusqu'à ce qu'il se produise une nette diminution de la formation de méthanol dans la zone de réaction, on interrompt la réaction et on purge ensuite le catalyseur, immédiatement après l'interruption de la réaction, avec des gaz inertes à des températures de 10 à 300°C et on le régénère in situ, à la pression normale ou à une pression modérément élevée, en faisant passer sur le catalyseur des gaz contenant de l'oxygène à des températures de 150 à 200°C, la teneur en oxygène pendant tout le déroulement de la régénération étant adaptée à la température particulière et le passage des gaz contenant de l'oxygène étant poursuivi jusqu'à ce que le maximum du profil des températures soit passé par la totalité du catalyseur.